Europäisches Patentamt

(19) European Patent Office

Office européen des brevets

(11) **EP 0 782 450 B1**

(12) **EUROPÄISCHE PATENTSCHRIFT**

(45) Veröffentlichungstag und Bekanntmachung des Hinweises auf die Patenterteilung:
**04.06.2003 Patentblatt 2003/23**

(51) Int Cl.⁷: **A61K 31/565**, A61K 31/57, A61K 31/585, A61P 5/32

(21) Anmeldenummer: **95934094.4**

(22) Anmeldetag: **22.09.1995**

(86) Internationale Anmeldenummer:
**PCT/EP95/03733**

(87) Internationale Veröffentlichungsnummer:
**WO 96/009057 (28.03.1996 Gazette 1996/14)**

(54) **VERWENDUNG VON AROMATASEHEMMERN ZUR HERSTELLUNG EINES ARZNEIMITTELS ZUR BEHANDLUNG EINES RELATIVEN ANDROGENMANGELS BEIM MANN**

USE OF AROMATASE INHIBITORS IN THE PREPARATION OF A DRUG FOR TREATING A RELATIVE ANDROGEN DEFICIENCY IN MEN

UTILISATION D'INHIBITEURS D'AROMATASE POUR LA PRODUCTION D'UN MEDICAMENT DESTINE AU TRAITEMENT D'UNE DEFICIENCE RELATIVE D'ANDROGENE CHEZ L'HOMME

(84) Benannte Vertragsstaaten:
**AT BE CH DE DK ES FR GB GR IE IT LI LU MC NL PT SE**

(30) Priorität: **22.09.1994 DE 4435368**

(43) Veröffentlichungstag der Anmeldung:
**09.07.1997 Patentblatt 1997/28**

(73) Patentinhaber: SCHERING AKTIENGESELLSCHAFT
**13353 Berlin (DE)**

(72) Erfinder:
 • **RADLMAIER, Albert**
 **D-16727 Berlin (DE)**
 • **HABENICHT, Ursula-Friederike**
 **D-14167 Berlin (DE)**
 • **NEUMANN, Friedmund**
 **D-14089 Berlin (DE)**

(56) Entgegenhaltungen:
 • **EPILEPSIA, 1991, 32 SUPPL 6 PS34-7, UNITED STATES, HERZOG AG 'Reproductive endocrine considerations and hormonal therapy for men with epilepsy.'**
 • **ANN MED, JUN 1993, 25 (3) P235-41, FINLAND, DE LIGNIERES B 'Transdermal dihydrotestosterone treatment of 'andropause'.'**
 • **THE WESTERN JOURNAL OF MEDICINE, Bd. 159, Nr. 5, November 1993 Seite 618-20 MATSUMOTO, A. M. '"Andropause"- are reduced androgen levels in aging men physiologically important?'**
 • **JOURNAL OF CLINICAL ENDOCRINOLOGY & METABOLISM, 78 (3). 1994. 711-716., BAGATELL C J ET AL 'Effects of endogenous testosterone and estradiol on sexual behavior in normal young men'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft die neue Verwendung von selektiven Aromatasehemmern zur Herstellung eines Arzneimittels zur Behandlung eines relativen Androgenmangels beim Mann.

Mit zunehmendem Alter kommt es beim Mann zu einer Abnahme der testikulären Androgenproduktion und der Androgenkonzentration im Organismus. Im Gegensatz zur Situation bei der Frau, in der die Östrogenproduktion innerhalb eines vergleichsweise kurzen Zeitraums auf Kastrationswerte abfällt, handelt es sich beim Mann um ein über Jahrzehnte dauerndes Geschehen und einen nur graduellen Abfall. Es läßt sich dennoch eindeutig nachweisen, daß die Gesamtkonzentration von Testosteron im Serum in der höheren Altersgruppe signifikant gegenüber den Werten bei jungen Männern erniedrigt ist. Durch den gleichzeitig mit dem Alterungsprozeß einhergehenden Anstieg des steroidhormonbindenden Globulins (SHBG) sinkt zudem der Anteil des freien, nicht gebundenen und somit biologisch wirksamen Testosteron. Weiterhin fällt auf, daß die Serumspiegcl der Östrogene, obwohl sie durch direkte Umwandlung aus Androgenen entstehen, nicht in gleicher Weise altersabhängig absinken. Dadurch wird das hormonelle Milieu deutlich verschoben.

Beim Mann ist das hormonelle Milieu der Sexualsteroide gekennzeichnet durch ein deutliches Übergewicht der Androgene gegenüber den Östrogenen. Während die zirkulierende Hauptkomponente der Androgene, Testosteron, im Serum in Einheiten im Bereich von nmol/l nachgewiesen wird, wird der Östrogen-Gegenspieler, Östradiol, nur im Bereich von pmol/l gemessen. Dieses deutliche Übergewicht der Androgene ist grundsätzlich in der gesamten nachpubertären Lebensspanne nachweisbar, jedoch zeigt sich eine deutlich unterschiedliche Ausprägung dieser Androgendominanz in Abhängigkeit vom Lebensalter. Mit zunehmendem Alter, und deutlicher ausgeprägt bei den über 60jährigen, findet sich eine geringer ausgeprägte Betonung des Androgen-Übergewichts. Publizierte Meßserien, in denen die Relation Serum Tesiosteron zu Serum Östradiol im Vergleich von jungen zu älteren ($\geqslant$ 60. Lebensjahr) Männern bestimmt wurde, zeigt Tabelle 1.

Tabelle 1

| Vergleich des $T/E_2$-Verhältnisses im Serum bei jungen und älteren Männern | | | |
|---|---|---|---|
| **Referenz** | **Jung (< 60 J.)** | **Älter (> 60 J.)** | **% $\triangle$ (Abnahme)** |
| Deslypere et al. [1] | 206:1 | 128:1 | - 38 % |
| Pirke & Dörr [2] | 324:1 | 174:1 | - 46 % |
| Baker et al. [3] | 372:1 | 225:1 | - 31 % |
| Murano et al. [4] a.m. p.m. | 155:1 160:1 | 98:1 84:1 | -37% -48% |

1) Deslypere, J. P. et al., Journal of Clinical Endocrinology and Metabolism, 64, No. 1, 1987

2) Pirke, K. M. & Doerr, P., Acta Endocrinologica, 74 (1973), 792-800

3) Baker, H. W. G. et al., Clinical Endocrinology, 5 (1976), 349-372

4) Murano, E. P. et al., Acta Endocrinologica, 99 (1982), 619-623

Obwohl in den erwähnten Arbeiten das Verhältnis Testosteron zu Östradiol teilweise in deutlich unterschiedlichen Größenordnungen angegeben wird - was auf die verwendeten unterschiedlichen Meßmethoden zurückzuführen ist - zeigt sich bei den älteren Männern eine auffallende Übereinstimmung in den aufgefundenen relativen Abnahmen des Übergewichts von Testosteron um 30 - 50 % gegenüber dem Vorwert bei den jungen Männern.

Das entstehende relative Testosterondefizit kann sich in vielerlei Hinsicht ungünstig auswirken. So wird z.B. vermutet, daß ein durch den Testosteronabfall, bei insgesamt etwa gleichbleibenden Östrogenkonzentrationen, einhergehendes Ungleichgewicht zwischen Androgenen und Östrogenen für die Entstehung der benignen Prostatahyperplasie (BPH) von entscheidender Bedeutung ist. Unabhängig von den Wirkungen der Östrogene, kann allerdings auch das relative Testosterondefizit per se für eine Reihe von altersbedingten Störungen verantwortlich gemacht werden. Zu nennen ist hier die Abnahme der Muskelmasse, einhergehend mit Einschränkung der körperlichen Leistungsfähigkeit, Abnahme der Knochendichte, in Einzelfällen bis zur Osteoporose, Reduktion von Libido und Potenz und psycho-vegetative Störungen. Alle genannten Störungen werden oft unter der Bezeichnung Klimakterium virile zusammengefaßt.

Die gängige Behandlung dieses mutmaßlich durch Androgenmangel bedingten Syndroms besteht bisher in der exogenen Zufuhr von Androgenen. Zum Einsatz kommen oral wirksame Androgene und intramuskulär zu applizierende langkettige Testosteronester mit Depoteffekt. Diese Therapieformen sind geeignet, die androgenmangelbedingten Symptome zu bessern, stellen allerdings nur eine unzureichende Annäherung an den physiologischen Zustand her.

Als oral zu verabreichende Substanz wird entweder ein Testosteronderivat, also kein natürliches Testosteron, zugeführt (z.B. Proviron®) oder die Verabreichung geht mit einem von der physiologischen Situation abweichenden überproportionalen Anstieg von Dihydrotestosteron (DHT) einher (z.B. Andriol®). DHT scheint im Gegensatz zu Testosteron der Androgenbestandteil zu sein, der für die Entwicklung der BPH und auch der androgenetischen Alopezie von großer Bedeutung ist.

Bei den Depotformulierungen stellt die ungleichmäßige Freisetzung aus dem Depot ein bisher noch nicht befriedigend gelöstes Problem dar; initial kommt es zu einem deutlich über den Normalbereich hinausreichenden Testosteronanstieg, gegen Ende des Dosierungsintervalls dagegen zu deutlich erniedrigten Testosteronwerten.

Seit langem ist bekannt, daß an dem endokrinen Regelkreis, durch den beim Mann die Androgenspiegel auf konstantem Niveau gehalten werden, neben den Androgenen auch Östrogene beteiligt sind. Durch Gabe pharmakologischer Dosen östrogenwirksamer Substanzen, wie z.B. Diethylstilbestrol, gelingt es bei Patienten mit Prostatakarzinom, die hypophysäre LH-Freisetzung weitgehend zu unterdrücken und die Testosteronspiegel im Serum auf Kastrationsniveau zu senken.

Aus den Erfahrungen mit dem Einsatz von reinen Antiandrogenen bei Patienten mit Prostatakarzinom, die der gleichen Altersgruppe angehören wie Patienten mit Klimakterium virile, läßt sich das Ausmaß des gegenregulatorischen Potentials abschätzen. Wird durch reine Antiandrogene wie Flutamid oder Casodex die zentrale Hemmwirkung von Androgenen unterbunden, so kommt es in dieser Altersgruppe zu einem gegenregulatorischen Anstieg der Serumtestosteronkonzentration um ca. 50 - 60 % gegenüber dem Ausgangswert. Bei einer über Monate andauemden Behandlung zeigten sich allerdings bei den mit reinen Antiandrogenen behandelten Prostalakarzinom-Palienten Hinweise auf ein Nachlassen der Aktivität der Gegenregulation, d.h. die initial deutlich erhöhten Androgenspiegel fallen wieder ab (Lund und Rasmussen, 1988; Mahler und Denis, 1990; Delaere und Van Thillo, 1991).

Auffallend ist, daß die Abnahme der Androgene im Alter nicht über eine Aktivierung des Gegenregulationsmechanismus verhindert wird. Als Ursache hierfür gilt, daß zum einen mit dem Alter die Hodenfunktion allgemein nachläßt, zum anderen aber auch der Feedback-Mechanismus eine gesteigerte Sensitivität für Sexualsteroide aufweist (Deslypere, J. P. et al., Journal of Clinical Endocrinology and Metabolism, 64, No. 1, 1987). Folglich ist davon auszugehen, daß bei älteren Männern im Vergleich mit jüngeren (siehe unten) eine geringer ausgeprägte Gegenregulation vorliegt, weshalb für die langfristige Anwendung mit keiner gegenüber dem Ausgangswert erhöhten Serumandrogenkonzentration zu rechnen ist.

Demgegenüber ist bekannt, daß bei jüngeren Männern durch tägliche Behandlung mit Antiöstrogenen (mit jeweils erheblicher östrogener Partialwirkung), die Testosteronwerte auch in einer Langzeitbehandlung nachhallig erhöhl werden (Treatment of Male Infertility, Springer-Verlag Berlin, Heidelberg, New York 1982; Fuse, H. et al., Archives of Andrology 31 (1993) 139-145).

Antiöstrogene scheinen aufgrund theoretischer Erwägungen zur Behandlung eines relativen Androgenmangels beim Mann wenig geeignet. So hat eine Behandlung mit Antiöstrogenen keinen Einfluß auf den Östrogenspiegel, da diese die Wirkung der Östrogene an deren Rezeptor blockieren. Bei Verwendung von Antiöstrogenen als Rezeptorenblocker führte eine ungenügende Compliance sofort zum ungünstigsten Effekt, da wegen der einsetzenden Gegenregulation die höhere Östrogenkonzentration unmittelbar über die nun freigewordenen Rezeptoren wirken kann.

Ein weiterer Nachteil einer Antiöstrogen-Behandlung ist die Ungewißheit, ob die Blockade der Östrogen-Rezeptoren in allen östrogenabhängigen Geweben und Organen gleich stark ausgeprägt ist und welche Bedeutung die Eigenöstrogenität, wie sie z.B. das bekannteste Antiöstrogen Tamoxifen aufweist, für die Anwendung beim Mann hat.

Die vorliegende Erfindung hat die Aufgabe, geeignete Substanzen bereitzustellen, die einen relativen Androgenmangel beim Mann bei gleichzeitiger Annäherung an das physiologische hormonale Verhältnis von Androgcnen zu Östrogenen beheben und die die genannten Nachteile vermeiden.

Diese Aufgabe wird gcmäß vorliegender Erfindung durch die Verwendung mindestens eines Aromatasehemmers zur Herstellung eines Arzneimittels zur Behandlung eines relativen Androgenmangels beim Mann gelöst.

Es wurde festgestellt, daß der Einsatz von Aromatasehemmern bei der Behandlung eines relativen Androgenmangels bei älteren Männern überraschenderweise zu einer langfristigen Erhöhung der Androgenspiegel führt.

Durch graduelle Absenkung der Östrogen-Konzentration wird eine gegenregulatorische Stimulierung der Androgensynthese induziert. Die Aromatasehemmer führen zu einer endogenen Rebalancierung des Testosteron-/Östrogenverhältnisses beim Mann; dadurch wird der relative Androgenmangel wieder ausgeglichen.

Vorzugsweise Zur Herstellung eines Arzneimittels zur Behandlung eines relativen Androgenmangels beim Mann, werden gemäß vorliegender Erfindung selektive Aromatasehemmer verwendet. Unter selektiven Aromatasehemmern sollen solche Verbindungen verstanden werden, die als Substrat für die Aromatase fungieren und in der eingesetzten Dosierung außer der Aromatase keine anderen Enzyme in klinisch relevanter Weise beeinflußen.

Als typische selektive Aromatasehemmer gemäß vorliegender Erfindung gelten beispielsweise die steroidalen Verbindungen

1-Methyl-androsta-1,4-dien-3,17-dion (DE-A 33 22 285; Atamestan),

4-Hydroxy-4-androsten-3,17-dion (Formestan)

sowie die nicht-steroidalen Aromalasehemmer

(RS)-5 (4-Cyanphenyl)-5,6, 7,8-tetrahydro-imidazo-(1,5α)-pyridin, Hydrochlorid (Cancer Res., 48, S. 834-838,1988; Fadrozol),

4-[Cyan-α-(1,2,4-triazol-1-yl)-benzyl]-benzonitril (CGS 20267),

5-[Cyclopentyliden-(1-imidazolyl)-methyl ]-thiophen-2-carbonitril (EP-A 0 411 735; Pentrozol),

2,2'-[5-(1H',2',4-Triazol-1-yl-methyl)-1,3-phenylen]-bis(2'-methylpropionitril) (Arimidex) sowie

(6-[1-(4-Chlorphenyl)-1,2,4-triazol-1-yl)-methyl]-1-methyl-1H-benzotriazol, Dihydrochlorid (vorozol).

Die vorstehende Aufzählung selektiver Aromatasehemer ist nicht abschließend; auch andere, in den genannten Druckschriften und Publikationen beschriebenen Verbindungen sowie alle anderen Verbindungen, die den gestellten Forderungen genügen, kommen in Betracht.

Entgegen der Annahme, daß bei Behandlung älterer Männer mit einem Aromatasehemmer über mehrere Monate der gegenregulatorische Impuls nachlassen könnte, zeigen Daten aus länger dauernden Studien, z.B. mit Atamestan an Patienten mit BPH, daß auch nach 24 bis 48 Wochen langer Behandlung weiterhin eine signifikante Erhöhung der Testosteronkonzentration vorliegt.

Die entsprechenden Ergebnisse einer 24wöchigen, vierarmigen Studie im Vergleich zu Placebo (100 mg/d, 300 mg/d und 600 mg/d) zeigt Tabelle 2.

Tabelle 2:

| Testosteronserumkonzentration (ng/ml) unter Atamestan | | | |
|---|---|---|---|
| Tagesdosis | Vorwert | nach 24 Wochen | Δ % (Median ± SD) |
| Placebo | 4.13 | 4.18 | 6.00 ± 27.64 |
| 100 mg | 4.41 | 5.57 | 29.57 ± 34.70 |
| 300mg | 4.50 | 6.15 | 40.88 ±156.12 |
| 600 mg | 3.78 | 5.40 | 41.19 ± 37.62 |

Tabellen 3 und 4 zeigen die Ergebnisse nach einer 48wöchigen Behandlung.

Tabelle 3:

| Testosteronserumkonzentration (ng/ml) unter Atamestan | | | |
|---|---|---|---|
| Tagesdosis | Vorwert | nach 48 Wochen | Δ % (X ± SD) |
| Placebo | 4,6 | 4,1 | - 0,1 ± 43,1 |
| 400 mg | 4,2 | 5,4 | 42,9 ± 53,5 |

Tabelle 4:

| Testosteronserumkonzentration (ng/ml) unter Atamestan | | | |
|---|---|---|---|
| Tagesdosis | Vorwert | nach 48 Wochen | Δ % (X ± SD) |
| Placebo | 4,6 | 4,6 | 2,8 ± 26,9 |
| 100 mg | 5,1 | 5,9 | 19,0 ±36,8 |
| 300 mg | 4,7 | 6,6 | 41,7 ± 46,4 |

Durch graduelle Absenkung der Östrogenkonzentration wird eine gegenregulatorische Stimulierung der Androgensynthese induziert. Gewissermaßen kommt es zu einer endogenen Testosteronsubstitution, wodurch die Androgen-/Östrogen-Balance wieder in den "jugendlichen" Bereich zurückgeführt wird. Dies belegen die Ergebnisse der längerfristigen Behandlung älterer Männer (Durchschnittsalter über 60 Jahre) mit dem selektiven Aromalasehemmer Atamestan.

In mehreren klinischen Studien wurde Männern dieser Altersgruppe zur Behandlung einer bestehenden BPH Atamestan in unterschiedlichen Dosierungen und über Zeiträume bis zu 48 Wochen verabreicht. Die Ergebnisse zeigen, daß unter Atamestan-Behandlung für Patientenkollektive eine signifikante Verschiebung des Testosteron-/Östradiol-Verhältnisses zugunsten von Testosteron eintritt. Tabelle 5 gibt die Testosteron-/Östrogen-Ratio vor und nach Atamesian-Gabe für Patientenkollektive aus 4 Studien und 7 Behandlungsgruppen wieder.

Tabelle 5

| Änderungen des T/E$_2$-Verhältnisses unter Atamestan | | | |
|---|---|---|---|
| Behandlungsgruppe | Vorwert | Behandlung (Zeitpunkt) | % Δ |
| 100 mg | 248:1 | 418:1 (48. Woche) | + 41 % |
| 300 mg | 236:1 | 440:1 (48. Woche) | + 46 % |
| 400 mg | 207:1 | 454:1 (48. Woche) | +54% % |
| 200 mg t.i.d. | 116:1 | 214:1 (8. Woche) | + 46 % |
| 100 mg | 196:1 | 376:1 (24. Woche) | + 48 % |
| 300 mg | 199:1 | 473:1 (24: Woche) | + 58 % |
| 600 mg | 170:1 | 439:1 (24. Woche) | + 61 % |

Die Atamestangabe führt durchweg zu einer Neueinstellung der Testosteron/Östradiol-Balance zugunsten der androgenen Komponente. Diese Wirkung war über den gesamten Beobachtungszeitraum bis zu maximal 48 Wochen Behandlung nachweisbar. Obwohl die periphere Östrogen-Absenkung in den täglichen Dosierungen 100 mg-600 mg gleich stark ausgeprägt war, zeigt sich ein Trend zur stärkeren Betonung des androgenen Anteils bei hohen Dosierungen.

Unter der Annahme, daß die in der Altersgruppe der Patienten über 60 Jahren gegenüber den jüngeren Jahrgängen um 30 - 50 % erniedrigte Testosteron-Dominanz (relativer Androgenmangel) das Beschwerdebild des "männlichen Klimakteriums" verursacht, ist somit das Ziel, das ursprüngliche "Kräfteverhältnis" zwischen Androgenen und Östrogenen wiederherzustellen, durch die Gabe eines, vorzugsweise selektiven, Aromatasehemmers durch Stimulation der endogenen Testosteron-Substitution ohne Notwendigkeit einer exogenen Hormonzufuhr erreicht. Anhand der Maßgabe, daß die im Alter vorherrschende Relation das Ergebnis einer 30 - 50 %igen Absenkung gegenüber den jugendlichen Werten ist, d.h. immer noch 50 - 70 % des Vorwertes beträgt, läßt sich für jeden einzelnen Patienten der entsprechende "jugendliche" Vorwert kalkulieren. Ein 70jähriger Patient mit einem Testosteron-/Östradiol-Verhältnis von 230:1 muß demnach auf eine neue Balance im Bereich zwischen 229:1 bis 460:1 eingestellt werden, damit eine vorangegangene 30- bzw. 50 %ige Abnahme ausgeglichen ist. Tabelle 6 zeigt das Ergebnis einer derartigen Kalkulation für die Patientenkollektive der in Tabelle 4 aufgeführten Atamestan-Studien.

Tabelle 6

| Vergleich zwischen kalkuliertem "jugendlichen" T/E$_2$-Bereich und gemessenen Werten unter verschiedenen Atamestan-Tagesdosierungen | | | |
|---|---|---|---|
| Tagesdosis | Vorwert | Kalkulierter Zielbereich zum Ausgleich einer 30-50 % igen Absenkung | Erreichter Wert unter Atamestan |
| 100 mg | 248:1 | 354: 1↦ 496:1 | 418:1 |
| 300 mg | 236:1 | 337:1 ↦ 472:1 | 440:1 |
| 400 mg | 207:1 | 296:1 → 414:1 | 454:1 |
| 100 mg | 196:1 | 280:1 ↦ 392:1 | 376:1 |
| 300 mg | 199:1 | 284:1 ↦ 398:1 | 473:1 |
| 600mg | 170:1 | 243:1 ↦ 340:1 | 439:1 |

Mit einer Tagesdosis von 100 mg ist der Zielberceich im allgemeinen gut getroffen. Bei höheren Dosierungen hingegen liegt das Ergebnis etwas oberhalb des Zielbereichs.

Durch Messung der Serumkonzentration von Testosteron und Östradiol kann somit frühzeitig kontrolliert werden, ob die angestrebte Hormonbalance erreicht wurde und gegebenenfalls eine Dosisanpassung vorgenommen werden.

Im allgemeinen werden täglich 25 bis 1000 mg, vorzugsweise 50 bis 600 mg, Atamestan oder eine biologisch äquieffektive Menge eines anderen Aromatasehemmers zur Behandlung eines relativen Androgenmangels beim Mann eingesetzt.

Die Aromatasehemmer können z. B. oral oder parenteral appliziert werden.

Für die bevorzugte orale Applikation kommen insbesondere Tabletten, Dragèes, Kapseln, Pillen, Suspensionen oder Lösungen infrage, die in üblicher und dem Fachmann geläufiger Weise mit den in der Galenik für die Formulierung -von oral zu applizierenden Aromatasehemmern üblichen Zusätzen und Trägersubstanzen hergestellt werden können.

Das erfindungsgemäß hergestellte Arzneimittel enthält als Wirkstoff je Dosiseinheit den Aromatasehemmer Atamestan in einer Dosierung von 50 bis 500 mg neben den üblichen Hilfs-, Träger- und/oder Verdünnungsmitteln oder andere Aromatasehemmer in biologisch äquieffektiver Dosierung.

Ein typische Zusammensetzung für eine Formulierung des Aromatasehemmers Atamestan als Tablette ist im nachstehenden Beispiel wiedergegeben:

**Beispiel**

| | |
|---|---|
| 100,0 mg | 1-Methyl-androsta-1,4-dien-3,17-dion |
| 140,0 mg | Laktose |
| 70,0 mg | Maisstärke |
| 2,5 mg | Poly-N-Vinylpyrrolidon 25 |
| 2,0 mg | Aerosil |
| 0.5 mg | Magnesiumstearat |
| 315,0 mg | Gesamtgewicht der Tablettc, die in üblicher Weise auf einer Tablettenpresse hergestellt wird. |

Bei Verwendung von Aromatasehemmern zur Behandlung des Klimakterium virile wird die Ostrogenkonzentration wirkungsvoll gesenkt. Die leichte Steuerbarkeit der Therapie zeichnet die Behandlung mit einem Aromatasehemmer zur Stimulation der endogenen Testosteronproduktion gegenüber dem Einsatz mit Antiöstrogenen aus. Wie bereits ausgeführt, ist mit Antiöstrogenen eine prospektive Steuerung der Therapie durch frühzeitige Messung pharmakodynamischer Parameter nicht möglich.

**Patentansprüche**

1. Verwendung mindestens eines selektiven Aromatasehemmers zur Herstellung eines Arzneimittels zur Behandlung eines relativen Androgenmangels beim Mann.

2. Verwendung von Atamestan, Formestan, Pentrozol, Arimidex, Fadrozol, CGS 20267, Vorozol nach Anspruch 1.

**Claims**

1. Use of at least one selective aromatase inhibitor for the production of a pharmaceutical agent for treating a relative androgen deficiency in men.

2. Use according to claim 1 of atamestane, formestane, pentrozole, arimidex, fadrozole, CGS 20267 or vorozole.

**Revendications**

1. Utilisation d'au moins un inhibiteur sélectif d'aromatase pour la production d'un médicament destiné au traitement d'une déficience relative d'androgène chez l'homme.

2. Utilisation d'atamestane, de formestane, de pentrozole, d'Arimidex, de fadrozole, de CGS 20267, de vorozole selon la revendication 1.